# EUROPEAN PATENT SPECIFICATION

(11) **EP 2 923 684 B1**
(45) Date of publication and mention of the grant of the patent: **14.02.2018**
(21) Application number: 15160699.3
(22) Date of filing: 25.03.2015
(51) Int. Cl.: A61H 1/02, A61F 5/01, A47C 20/00, A61G 7/075

(54) **CUSHION FOR ALLEVIATING BACK PAIN**
KISSEN ZUR LINDERUNG VON RÜCKENSCHMERZEN
COUSSIN PERMETTANT DE SOULAGER LE MAL DE DOS

(30) Priority: 27.03.2014 BE 201400207
(43) Date of publication of application: 30.09.2015
(73) Proprietor: 's Heeren, Katrien Justina, 1851 Grimbergen (BE)
(72) Inventor: 's Heeren, Katrien Justina, 1851 Grimbergen (BE)
(74) Representative: Philippaerts, Yannick

(56) References cited:
- DE-U1- 29 802 386
- US-A- 2 785 418
- US-A- 3 333 286
- US-A- 5 097 553
- US-A1- 2004 226 096
- Anonymous: "Vitacare Medical Products", , 9 December 2013 (2013-12-09), pages 1-9, XP055155629, Concord, Canada Retrieved from the Internet: URL:https://web.archive.org/web/2013120904 3734/http://vitacaremedical.com/product.ph p?id_category=19 [retrieved on 2014-11-28]

## Description

The invention relates to a cushion for alleviating pain in a lower back and pelvis of a user.

Pain in the lower back occurs frequently in the case of people with back disorders. People without back disorders can also experience pain in the lower back due to incorrect or heavy loading of the back, for instance during a pregnancy. It is generally known to the medical profession that pain in the lower back can be alleviated by taking up a lying position, wherein the legs are supported in an upward angled position. A cushion (often formed in practice by a stack of pillows) is then typically laid under the legs such that the upper legs extend upward and the lower legs extend in substantially lying position (so as to thus obtain the angled upper position). The legs are in this way as it were raised while the user lies on his/her back. Vertebrae in the lower back are hereby relieved of load. A drawback of supporting the legs by means of a stack of pillows is that the legs are not supported in stable manner. The pelvic floor muscles will hereby exert a force instead of relaxing since the pelvis functions as point of attachment for the stomach muscles. The pelvis is connected via joints to the spinal column and the two thighbones.

DE29802386 describes a support cushion for alleviating pain in a back of a user according to the preamble of claim 1. US 5,097,553 describes a support cushion for supporting the legs for the purpose of relieving pain in a lower back. Because this cushion is specifically shaped to support the lower legs, the legs will be supported in more stable manner than if they lie on a stack of pillows. A drawback of the support cushion of US 5,097,553 is that support is not optimal for the purpose of obtaining an ideal lying position for relieving pain in the lower back.

US 6,117,095 also describes a cushion for supporting the legs and the lower back for the purpose of relieving pain in the lower back. The portion of the cushion which supports the legs is fixed here to the portion of the cushion which supports the back. This improves the stability of the support. A drawback of the support cushion of US 6,117,095 is that inflation of the cushion detracts considerably from the convenience of use. The position of the user will also depend on the extent to which the cushion is inflated. Theoretically the cushion can hereby support the user in a proper position, but in practice this will depend on the amount of air blown into the cushion. As a result this cushion in practice rarely supports the user in an ideal and stable position.

It is an object of the invention to provide a cushion for alleviating pain in the lower back of the user, wherein the cushion is provided so as to support a user in an optimal position.

The invention provides for this purpose a cushion for alleviating pain in a lower back of a user, wherein the cushion comprises a first segment and a second segment which are connected to each other and which are formed such that, when the user lies on the cushion, the first segment supports at least the lower back and the pelvis of the user and the second segment supports the legs of the user such that the upper legs extend upward and the lower legs extend in lying position, wherein the second segment has an upper surface with gutter-shaped recesses which, when the user is lying on the cushion, extend at the position of the lower legs in order to hold the lower legs at a predetermined distance from each other.

The cushion according to the invention comprises different technical features which co-act synergistically to support the user in an ideal position. The first segment and the second segment are on the one hand connected to each other. As a result the second segment cannot be pushed away from the first segment when the legs rest on the second segment. Due to the angle of the upper legs relative to the lower legs and due to the angle of the upper legs relative to the upper body (the user lies on the cushion while the upper legs extend upward), the upper legs will always tend to push the second segment away from the upper body of the user. However, because the second segment is connected to the first segment and because the first segment supports the lower back of the user (and is therefore positioned fixedly relative to the upper body of the user), sliding away of the second segment is prevented such that the legs, at least in respect of the angle relative to upper body and lower legs, can be held fixedly in an optimal position. The lower legs are nestled in the cushion. Tests have further shown that the distance between the lower legs is crucial in enabling proper relaxation of the lower back and the pelvis and in alleviating the pain in the lower back. The distance between the lower legs can be predetermined by providing gutter-shaped recesses in an upper surface of the second segment. Because the lower legs lie embedded/nestled in the respective channels, a tension is exerted by means of gravitational force via the knees and the upper legs of the user on the pelvic floor muscles so that pain in the lower back of the user is alleviated in an efficient manner.

The first segment preferably has a preformed indentation in a longitudinal direction and in a transverse direction such that, when the user lies on the cushion, the lower back is held in the indentation. Owing to the preformed indentation, and in particular the nestling of the lower back and pelvis therein, the lower back will always have a predetermined stable position on the first segment. The lower back will hereby be positioned fixedly relative to the cushion such that the cushion also has less of a tendency to still slide away from the user (due to the forces of the legs exerted on the second segment as described above). Because the first segment and the second segment are connected, the positions of the gutter-shaped recesses will further be predetermined relative to the position of the indentation. Not only the spacing between the legs but also the relative position of the lower body and the lower legs will in this way be determined by the shape of the cushion. The body can hereby be held in a symmetrical position such that, due to the indentation and the recesses, the user will always come to lie in a predetermined symmetrical position on the cushion. This symmetrical position, which is obtained by the combination of the indentation and the gutter-shaped recess, is particularly suitable for alleviating pain in the lower back.

The indentation is preferably shaped such that a lower lumbar part of the back is supported ergonomically. Through the ergonomic support of a lower lumbar part of the back an optimum relief and relaxation of the lower back is obtained, whereby pain is alleviated in optimal manner.

The second segment preferably has an upward surface for supporting the upper legs, this upward surface extending between the first segment and the upper surface of the second segment. When the user lies on the cushion, the upper legs will rest against the upward surface. The upward surface therefore preferably has a length substantially equal to the length of the upper legs. The upward surface extends between the first segment and the upper surface of the second segment and prevents the upper surface of the second segment being pushed away from the first segment.

The upward surface preferably has an angle with a ground surface of about 90 degrees. Tests have shown that an angle of 90 degrees is optimal for alleviating pain in a lower back of a user. The upward surface more preferably also has an angle with the lying surface of about 90 degrees. Tests have shown that this further angle of 90 degrees is also optimal for alleviating pain in a lower back.

The first segment and the second segment are preferably manufactured integrally. When the first segment and the second segment are manufactured integrally, use of the cushion is further simplified because the segments no longer have to be attached to each other by the user. The chance of erroneous use of the cushion resulting from incorrect attachment of the first segment to the second segment is also reduced. When the first segment and the second segment are manufactured integrally as one entity, the second segment cannot be pushed away from the first segment when the user lies on the cushion.

The cushion is preferably manufactured from a foam material. Foam material is known in the manufacture of cushions and is particularly suitable for forming a cushion of a predetermined shape. The shape of the foam here determines the shape of the cushion.

The cushion preferably has a sleeve enclosing the first and the second segment. Even when the first segment and second segment are not manufactured integrally, the first segment and the second segment will nevertheless be connected to each other by the sleeve when the sleeve encloses the first and second segments. A further advantage of the sleeve is that the sleeve protects the first segment and the second segment (each manufactured for instance from foam) from soiling. The sleeve is preferably made here from a washable material.

The cushion preferably has at least one handgrip on a side of the cushion. Owing to the handgrip the user can manipulate the cushion in simple manner when the user wishes to lie on the cushion or when the user wishes to move off the cushion.

The second segment preferably further has reinforcing elements for supporting the legs. The reinforcing elements help to support the legs, in particular to bear the weight of the legs. Because of the reinforcing elements the foam can be given a lighter form, i.e. with a higher compressibility, without this having an adverse effect on the operation of the cushion. Using a lighter foam further increases the comfort of the cushion since a lighter foam is typically perceived by a user as being softer.

A heating element is preferably provided in the first segment for heating the lower back. The lower back can relax further due to the heat, whereby pain can be alleviated more efficiently.

The second segment preferably has a recess at the position of the hollow of the knee when the user is lying on the cushion. When upper legs and lower legs are placed at an angle of 90 degrees relative to each other, muscles and/or tendons are typically located in the hollow of the knee which round off the inner angle between upper legs and lower legs. Providing a recess on the second segment at the position of the hollow of the knee enables lower legs and upper legs to be placed at an angle of 90 degrees relative to each other without appreciable pressure points at the position of the hollow of the knee. This increases the comfort of the cushion.

The recesses are preferably positioned parallel with a centre-to-centre spacing of at least 10 cm, preferably at least 20 cm. The lower legs are hereby supported parallel to each other at a spacing of 10 cm, preferably at least 20 cm. Tests have shown that this position is optimal in alleviating pain in a lower back of a user.

The invention will now be further described on the basis of an exemplary embodiment shown in the drawing.

In the drawing:
figure 1 shows a side view of a user lying on a cushion according to the invention; and
figure 2 shows a side view and a cross-section of a cushion according to an embodiment of the invention.

The same or similar elements are designated in the drawing with the same reference numerals.

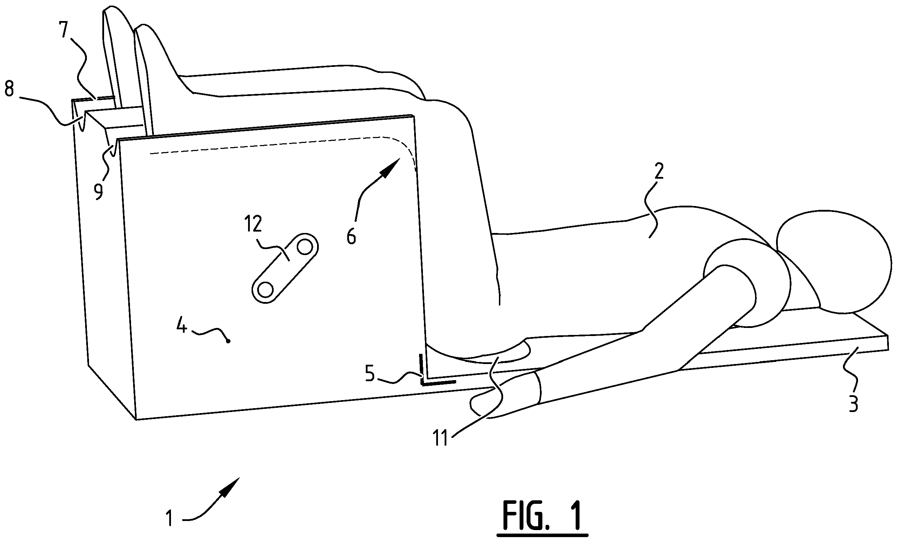

Figure 1 shows a perspective view of the cushion 1 according to an embodiment of the invention. Figure 1 shows here a user 2 lying on cushion 1. Lying on the cushion is defined as taking up a position wherein the user rests in a substantially horizontal position on a surface. Lying is preferably defined here as taking up a position wherein at least the upper body and the lower legs of the user lie substantially horizontally on a surface.

Cushion 1 according to the invention comprises technical features, which are described in further detail below, for the purpose of optimally supporting a user in a so-called psoas position. The psoas position is a position in which hips as well as knees form an angle of 90 degrees. In this position pressure is removed from vertebrae, bone structures, tendons and muscles in the lower back. This position is therefore very relaxing and highly suitable for alleviating pain in a lower back of a user.

Cushion 1 has a first segment 3 and a second segment 4. First segment 3 is connected to second segment 4. First segment 3 and second segment 4 are preferably manufactured integrally. If first segment 3 and second segment 4 are not manufactured integrally, they can be connected in different ways. First segment 3 can thus be adhered to second segment 4. First segment 3 can alternatively be connected via clamps to second segment 4. As a further alternative first segment 3 and a second segment 4 can be placed in a sleeve (also referred to as pillowcase), wherein the sleeve defines and maintains the relative position of first segment 3 and second segment 4. First segment 3 and second segment 4 are preferably manufactured from a foam material suitable for manufacturing cushions such as support cushions.

First segment 3 is intended for the purpose of supporting at least a lower back of user 2. In the embodiment shown in figure 1 first segment 3 is shaped so as to support the whole upper body (lower back, middle back and upper back) of the user. According to a further alternative embodiment, the whole upper body and the head of user 2 can be supported by first segment 3. First segment 3 preferably comprises a preformed indentation 11 at the position of the lower back. This indentation 11 is preferably formed in both the longitudinal direction and the transverse direction of cushion 1. Indentation 11, the nestling location for the lower back and the pelvis, functions as support for the optimum position when the user is lying on the cushion. The user lies with the pelvis in the indentation so that a permanent and stable angle is formed between the lower back and the upper legs. This nestling location also emphasizes that the product is an anatomically constructed cushion and thus follows the natural position of the lower back, particularly the lordosis or normal curvature of the lumbar vertebrae. Since the indentation is embedded into the cushion, the lower back of the user will take up a fixed stable position. Owing to the shape of the cushion the lower back of the user is hereby guided to an ideal position. This function of indentation 11 can also be referred to as nestling. The indentation 11 at the position of the lower back of user 2 has the further advantage that the lower back has at least partially lower support than other parts of the back (for instance the part of the lower back adjoining the middle back). The lower back is hereby supported in a naturally curved position. Tests have shown that this naturally curved position has a positive effect in relaxing the lower back, and hereby also has a positive effect in alleviating pain. Indentation 11 is preferably shaped here such that the lower back is supported in an ergonomic curvature. Indentation 11 has the further effect that it urges the upper legs of the user into the upward position and results in forming of the angle of 90 degrees with the upper body. First segment 3 of cushion 1 typically has a thickness of more than 5 cm, preferably more than 7 cm, more preferably more than 9 cm. The thickness of first segment 3 is further preferably less than 40 cm, more preferably less than 30 cm.

According to a preferred embodiment of the invention, first segment 3 is provided, for instance at the position of the indented recess 11, with heating means (not shown) for heating the lower back of the user. These heating means can be embodied in different ways. An electrical resistor can for instance thus be placed in or on the material of the cushion. A heat exchanger can alternatively be provided in or on the material of cushion, the heat exchanger then being provided for the purpose of generating heat to the lower back. A heating element is preferably provided in the first segment so that a transcutaneous electrical stimulation of the lower back takes place by means of heat. Due to the heat the lower back can relax optimally, whereby pain is alleviated.

Second segment 4 of cushion 1 has a thickness which is considerably greater than the thickness of first segment 3. The thickness of second segment 4 is ideally equal to the thickness of first segment 3 increased by the length of the upper leg of user 2. The skilled person will appreciate that different users also have different lengths of the upper legs and that this ideal thickness will therefore seldom be achieved in practice. The skilled person will however also appreciate that variations from this ideal thickness, provided these variations lie within determined limits, have little or no influence on the effect of cushion 1. The skilled person will therefore understand that the thickness of second segment 4 of cushion 1 is preferably more than 30 cm, according to another embodiment preferably more than 50 cm, and preferably less than 90 cm, and according to another embodiment preferably less than 65 cm. The skilled person will appreciate on the basis of the above explanation that the different thicknesses and thickness ratios of first segment 3 and second segment 4 are possible taking into account the anatomical characteristics of the intended user(s) 2 of cushion 1.

Second segment 4 has an upper surface 7 which preferably extends substantially horizontally. Upper surface 7 is formed for the purpose of supporting the lower legs of user 2. Upper surface 7 preferably has for this purpose two gutter-shaped recesses 8, 9. The two gutter-shaped recesses 8, 9 extend parallel to each other in a longitudinal direction of cushion 1 and are placed at a distance from each other. The centre-to-centre distance 10 between the gutter-shaped recesses is preferably more than 10 cm, more preferably more than 20 cm, most preferably more than 30 cm. The gutter-shaped recesses are more preferably placed symmetrically on cushion 1. The gutter-shaped recesses have the effect, when a user 2 is lying on cushion 1, that the lower legs of the user will tend as a result of gravitational force to sink to the deepest point of the gutter-shaped recesses (and remain there). The lower legs are thus held via the gutter-shaped recesses at a fixed position (and so also at a fixed relative position) by cushion 1. Tests have shown that the lower back of the user can relax considerably better when the lower legs are placed symmetrically at a distance from each other. A further advantage of the gutter-shaped recesses is that the user does not have to use any muscular strength to hold the lower legs in the ideal position, since the lower legs will remain lying in stable manner in this ideal position due to gravitational force. This fact enhances relaxing of the lower back and the pelvis, and consequently alleviation of pain in the lower back when a user lies on the cushion. Because the legs lie relaxed in the gutter-shaped recesses, the gravitational force will fulfil its function in respect of the lower back via the knees (which are folded at an angle of 90 degrees) and lower legs.

Second segment 4 further has an upward surface extending between upper surface 7 and first segment 3. This upward surface has an angle 5 of about 90 degrees with the upper surface of first segment 3. This upward surface further has at the position of upper surface 7 an angle 6 of about 90 degrees with upper surface 7. The upward surface hereby extends substantially vertically. The upward surface has a length (as measured in height direction of cushion 1) which is substantially equal to the length of the upper legs of user 2. The upward surface is suitable for supporting the upper legs in an upward position. It will be apparent here to the skilled person that, when user 2 is lying on cushion 1, the legs (and in particular the upper legs) will tend to move to a straightened position. The upper legs of user 2 will hereby push second segment 4 of cushion 1 away from first segment 3 of cushion 1. For this purpose the upper legs exert a force against the upward surface of second segment 4, this force being counteracted by the upward surface of second segment 4. Cushion 1, in particular second segment 4 of cushion 1, can be provided here with additional reinforcing elements for the purpose of counteracting this force of the upper legs. A reinforcing element can thus extend in the embodiment shown in figure 1 from a bottom left-hand side of the second segment (being a most distal lower angle of the second segment of the cushion) in the direction of the hollows of the knees of the user. The nestling of the lower back and the pelvis in indentation 11 will ensure that the upper legs lie in a good upward position so that only a minimal pressure is exerted against the upward surface of cushion. The skilled person will appreciate that alternative reinforcements can likewise be added to the cushion whereby the cushion can retain its shape in efficient manner.

Cushion 1 according to the invention is preferably provided on one or more of its lateral sides with a handgrip 12. Handgrip 12 is preferably placed here within reach of a user lying on the cushion. Handgrip 12 can hereby be used to remove the cushion when the user is lying on the cushion. Handgrip 12 can also be used to transport the cushion, for instance before or after use. When a handgrip is provided on either side of cushion 1, a belt can further be provided which extends from the one handgrip to the other such that the legs can be additionally pressed against the cushion. Children suffering from a spastic paralysis can also be assisted with this cushion when the carer can restrain the legs (with the belt) so that they do not fall onto the chest when they are lying on the cushion (the foetal position). This can also be used for people with symptoms of paralysis of the legs.

The angle between the upward surface and upper surface 7 of second segment 4 of cushion 1, at least at the position of the hollows of the knees of the user (when the user is lying on the cushion), preferably has not a sharp shape but a rounded shape. This angle between upper surface and upward surface is 90 degrees. At the position of the cavities of the knees of the user this angle is preferably rounded with a radius of a minimum of 1 cm, preferably a minimum of 3 cm, more preferably a minimum of 5 cm. This radius is used so as to provide space for the natural curvature of the popliteal space or hollow of the knee. Rounding the angle at the position of the hollows of the knees creates space for muscles and/or tendons present at the position of the hollow of the knee when the knee is bent. Unpleasant pressure points on the hollow of the knee are hereby avoided when the user lies on the cushion. As a further consequence the user will be able to form an angle of 90 degrees between upper legs and lower legs in comfortable manner. The roundings are preferably formed at the position of the gutter-shaped recesses, wherein the gutter-shaped recesses transpose via the radius of curvature to the upward surface.

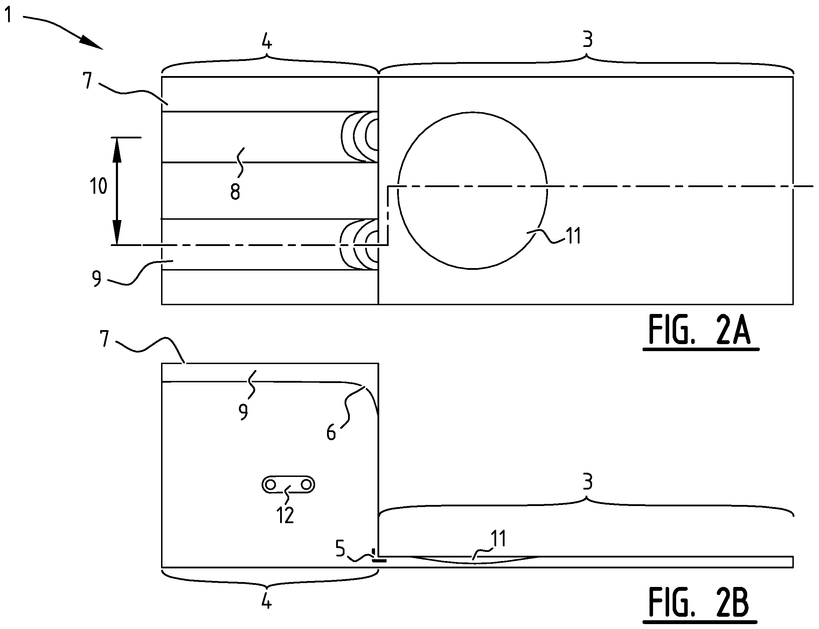

Figure 2 shows a top view and a corresponding side view of a cushion 1 according to the invention. The figure shows first segment 3 and second segment 4, wherein first segment 3 has an indented recess. The indented recess is positioned here at the location of the lower back of the user when the user lies on the cushion. This indented recess is preferably placed centrally in the transverse direction of cushion 1. Indented recess 11 is hereby symmetrical in the transverse direction of cushion 1. Figure 2 further shows second segment 4 of cushion 1 which has an upper surface 7 with two gutter-shaped recesses 8, 9. The gutter-shaped recesses 8, 9 are placed at a distance 10 from each other. The gutter-shaped recesses 8, 9 extend in a longitudinal direction of cushion 1. The gutter-shaped recesses 8, 9 are preferably placed symmetrically relative to the transverse direction of cushion 1. Figure 2 further shows how the gutter-shaped recesses are connected via a rounded angle 6 to the upward surface. In the embodiment as shown in figure 2 the rest of upper surface 7 is connected here via an acute angle (with negligible rounding) to the upward surface. The upward surface is further connected via an acute angle 5 to the upper surface of first segment 3. Figure 2 further shows a handgrip 12 on a side of cushion 1.

The cushion according to the invention can further be used to give disabled persons the possibility of taking up a body position other than their normal daily body position. In summary, the cushion can be used to alleviate back pain with support of the lower back and the pelvis by means of the psoas position.

A skilled person will understand on the basis of the above description how a cushion according to the invention can be formed in the different embodiments. It will be apparent here that the examples given are not limitative for the invention and that a number of alternatives are possible for achieving the effects of the invention. The invention will therefore be defined solely in the following claims.

## Claims

1. Cushion (1) for alleviating pain in a lower back and a pelvis of a user (2), wherein the cushion comprises a first segment (3) and a second segment (4) which are connected to each other and which are formed such that, when the user lies on the cushion, the second segment (4) supports legs of the user such that the upper legs extend upward and the lower legs extend in lying position, wherein the second segment has an upper surface with gutter-shaped recesses (8, 9) which, when the user is lying on the cushion, extend at the position of the lower legs in order to hold the lower legs at a predetermined distance from each other, **characterized in that** the first segment (3) supports at least the lower back of the user and wherein the first segment has a preformed indentation (11) in a longitudinal direction and in a transverse direction such that the lower back is held in the indentation when the user lies on the cushion.

2. Cushion (1) as claimed in claim 1, wherein the indentation (11) is shaped such that a lower lumbar part of the back is supported ergonomically.

3. Cushion (1) as claimed in any of the foregoing claims, wherein the second segment (4) has an upward surface for supporting the upper legs, this upward surface extending between the first segment and the upper surface of the second segment.

4. Cushion (1) as claimed in any of the foregoing claims, wherein the upward surface has an angle (5) with a ground surface of about 90 degrees.

5. Cushion (1) as claimed in claim 4, wherein the upward surface has an angle with an upper surface (7) of the second segment of about 90 degrees.

6. Cushion (1) as claimed in any of the foregoing claims, wherein the first segment (3) and the second segment (4) are manufactured integrally.

7. Cushion (1) as claimed in any of the foregoing claims, wherein the cushion is manufactured from a foam material.

8. Cushion (1) as claimed in any of the foregoing claims, wherein the cushion has a sleeve enclosing the first and the second segment.

9. Cushion (1) as claimed in any of the foregoing claims, wherein the cushion has at least one handgrip (12) on a side of the cushion.

10. Cushion (1) as claimed in any of the foregoing claims, wherein the second segment (4) further comprises reinforcing elements for supporting the legs.

11. Cushion (1) as claimed in any of the foregoing claims, wherein a heating element is provided in the first segment for heating the lower back.

12. Cushion (1) as claimed in any of the foregoing claims, wherein the second segment (4) has a recess (6) at the position of the hollow of the knee when the user is lying on the cushion.

13. Cushion (1) as claimed in any of the foregoing claims, wherein the gutter-shaped recesses (8, 9) are positioned parallel and have a centre-to-centre spacing of at least 10 centimetres, preferably at least 20 centimetres, more preferably at least 30 centimetres.

## Patentansprüche

1. Kissen (1) zur Linderung von Schmerzen im unteren Rücken und einem Becken eines Benutzers (2), wobei das Kissen ein erstes Segment (3) und ein zweites Segment (4) aufweist, die miteinander verbunden sind und die derart ausgebildet sind, dass das zweite Segment (4) die Beine des Benutzers trägt, so dass die Oberschenkel sich aufwärts erstrecken und die Unterschenkel sich in der Liegeposition erstrecken, wenn der Benutzer auf dem Kissen liegt, wobei das zweite Segment eine obere Oberfläche mit rinnenförmigen Aussparungen (8, 9) hat, die sich an der Position der Unterschenkel erstrecken, um die Unterschenke in einem vorgegebenen Abstand zueinander zu halten, wenn der Benutzer auf dem Kissen liegt, **dadurch gekennzeichnet, dass** das erste Segment (3) wenigstens den unteren Rücken des Benutzers trägt, und wobei das erste Segment eine vorausgebildete Einkerbung (11) in einer Längsrichtung und in einer Querrichtung hat, so dass der untere Rücken in der Einkerbung gehalten wird, wenn der Benutzer auf dem Kissen liegt.

2. Kissen (1) nach Anspruch 1, wobei die Einkerbung (11) derart geformt ist, dass ein unterer Lendenwirbelteil des Rückens ergonomisch getragen wird.

3. Kissen (1) nach einem der vorhergehenden Ansprüche, wobei das zweite Segment (4) eine ansteigende Oberfläche zum Tragen der Oberschenkel hat, wobei diese ansteigende Oberfläche sich zwischen dem ersten Segment und der oberen Oberfläche des zweiten Segments erstreckt.

4. Kissen (1) nach einem der vorhergehenden Ansprüche, wobei die ansteigende Oberfläche einen Winkel (5) mit einer Bodenoberfläche von etwa 90 Grad hat.

5. Kissen (1) nach Anspruch 4, wobei die ansteigende Oberfläche einen Winkel mit einer oberen Oberfläche (7) des zweiten Segments von etwa 90 Grad hat.

6. Kissen (1) nach einem der vorhergehenden Ansprüche, wobei das erste Segment (3) und das zweite Segment (4) integral gefertigt sind.

7. Kissen (1) nach einem der vorhergehenden Ansprüche, wobei das Kissen aus einem Schaumstoffmaterial hergestellt ist.

8. Kissen (1) nach einem der vorhergehenden Ansprüche, wobei das Kissen eine Hülle hat, die das erste und zweite Segment umschließt.

9. Kissen (1) nach einem der vorhergehenden Ansprüche, wobei das Kissen wenigstens einen Handgriff (12) auf einer Seite des Kissens hat.

10. Kissen (1) nach einem der vorhergehenden Ansprüche, wobei das zweite Segment (4) ferner Verstärkungselemente zum Tragen der Beine aufweist.

11. Kissen (1) nach einem der vorhergehenden Ansprüche, wobei in dem ersten Segment ein Heizelement zum Heizen des unteren Rückens bereitgestellt ist.

12. Kissen (1) nach einem der vorhergehenden Ansprüche, wobei das zweite Segment (4) eine Aussparung (6) an der Position der Kniekehle hat, wenn der Benutzer auf dem Kissen liegt.

13. Kissen (1) nach einem der vorhergehenden Ansprüche, wobei die rinnenförmigen Aussparungen (8, 9) parallel positioniert sind und einen Mitte-Mitte-Abstand von mindestens 10 Zentimetern, vorzugsweise mindestens 20 Zentimetern, noch bevorzugter mindestens 30 Zentimetern haben.

## Revendications

1. Coussin (1) pour soulager la douleur dans un bas de dos et un pelvis d'un utilisateur (2), dans lequel le coussin comprend un premier segment (3) et un second segment (4) reliés l'un à l'autre et formés de telle sorte que, lorsque l'utilisateur est allongé sur le coussin, le second segment (4) soutient les jambes de l'utilisateur de telle sorte que la partie supérieure des jambes s'étend vers le haut et la partie inférieure des jambes s'étend en position allongée, dans lequel le second segment a une surface supérieure comportant des creux en forme de gouttière (8, 9) qui, lorsque l'utilisateur est allongé sur le coussin, s'étendent au niveau de l'emplacement de la partie inférieure des jambes afin de garder la partie inférieure des jambes à une distance prédéterminée l'une de l'autre, **caractérisé en ce que** le premier segment (3) soutient au moins le bas du dos de l'utilisateur, et dans lequel le premier segment a une indentation préformée (11) dans une direction longitudinale et dans une direction transversale, de telle sorte que le bas du dos est maintenu dans l'indentation lorsque l'utilisateur est allongé sur le coussin.

2. Coussin (1) selon la revendication 1, dans lequel l'indentation (11) est formée de telle sorte qu'une partie lombaire inférieure du dos est soutenue de manière ergonomique.

3. Coussin (1) selon l'une quelconque des revendications précédentes, dans lequel le second segment (4) a une surface montante pour soutenir la partie supérieure des jambes, cette surface montante s'étendant entre le premier segment et la surface supérieure du second segment.

4. Coussin (1) selon l'une quelconque des revendications précédentes, dans lequel la surface montante présente un angle (5) ayant une surface au sol d'environ 90 degrés.

5. Coussin (1) selon la revendication 4, dans lequel la surface montante présente un angle ayant une surface supérieure (7) du second segment d'environ 90 degrés.

6. Coussin (1) selon l'une quelconque des revendications précédentes, dans lequel le premier segment (3) et le second segment (4) sont fabriqués d'une seule pièce.

7. Coussin (1) selon l'une quelconque des revendications précédentes, dans lequel le coussin est fabriqué à partir d'un matériau en mousse.

8. Coussin (1) selon l'une quelconque des revendications précédentes, dans lequel le coussin a une enveloppe renfermant le premier et le second segment.

9. Coussin (1) selon l'une quelconque des revendications précédentes, dans lequel le coussin a au moins une poignée (12) sur un côté du coussin.

10. Coussin (1) selon l'une quelconque des revendications précédentes, dans lequel le second segment (4) comprend en outre des éléments de renfort pour soutenir les jambes.

11. Coussin (1) selon l'une quelconque des revendications précédentes, dans lequel un élément de chauffage est prévu dans le premier segment pour chauffer le bas du dos.

12. Coussin (1) selon l'une quelconque des revendications précédentes, dans lequel le second segment (4) a un creux (6) au niveau de l'emplacement du creux du genou lorsque l'utilisateur est allongé sur le coussin.

13. Coussin (1) selon l'une quelconque des revendications précédentes, dans lequel les creux en forme de gouttière (8, 9) sont positionnés parallèlement et ont un espacement de centre à centre d'au moins 10 centimètres, de préférence d'au moins 20 centimètres, plus préférablement d'au moins 30 centimètres.
